# EUROPEAN PATENT SPECIFICATION

(11) **EP 1 814 505 B1**
(45) Date of publication and mention of the grant of the patent: **08.12.2010**
(21) Application number: 05796429.8
(22) Date of filing: 21.10.2005
(51) Int. Cl.: A61K 8/26, A61K 8/28, A61K 8/37, A61Q 15/00

(54) **UNDERARM COSMETIC METHOD**
KOSMETISCHES UNTERARM-VERFAHREN
PROCEDE COSMETIQUES POUR LES AISSELLES

(30) Priority: 26.11.2004 GB 0425945
(43) Date of publication of application: 08.08.2007
(73) Proprietor: Unilever PLC, London EC4Y 0DY (GB); Unilever N.V., 3013 AL Rotterdam (NL)
(72) Inventor: JAMES, Alexander, Gordon, Sharnbrook Bedfordshire MK44 1LQ (GB); MARTI, Vernon Peter J., Unilever R&D Port Sunlight, Bebington Merseyside CH63 3JW (GB); PATERSON, Sarah E., Unilever R & D Port Sunlight, Bebington Merseyside CH63 3JW (GB); POPLE, Jennifer, Elizabeth, Sharnbrook Bedfordshire MK44 1LQ (GB); TURNER, Graham Andrew, Unilever R&D Port Sunlight, Bebington Merseyside CH63 3JW (GB)
(74) Representative: Whaley, Christopher
(86) International application number: PCT/EP2005/011382
(87) International publication number: WO 2006/056283

(56) References cited:
- EP-A- 0 691 125
- EP-A- 1 428 521
- WO-A-02/102337
- US-A- 6 086 887

## Description

The present invention relates to a cosmetic method and more particularly to a method for simultaneously inhibiting or controlling underarm sweat and/or malodour and ameliorating or controlling skin darkening and compositions therefor.

### Background

In order to assist in regulating their body temperature, humans perspire, resulting in sweat remaining on the skin until it evaporates off. The extent to which this occurs depends on the density of sweat glands, the arm pit (axilla) being one area of the body where an especially high concentration occurs. Freshly perspired sweat on the skin is comparatively odourless, but the skin bacterial population can convert it to malodorous compounds, resulting the problem of body odour that many societies dislike or even abhor. Additionally, some societies dislike the presence of wet patches from sweat. Accordingly, a global industry has developed for controlling or inhibiting perspiration in limited regions of the body, such as in the underarm or other localised regions such as the soles of feet which are commonly, though not always, covered with clothing or footware and where bacterial populations can thrive on nutrient rich sweat.

Probably the most widely employed class of agents to control perspiration comprises astringent salts and especially astringent aluminium and/or zirconium salts. These agents act as a bactericidal deodorant thereby controlling bacterial numbers, and when sufficient sweat glands are blocked, act as an effective localised antiperspirant.

Compositions containing the aforementioned astringent salts have found considerable public favour for controlling odour and/or perspiration, but an additional problem has been identified. Some people exhibit a propensity for their skin to redden or darken following application of such compositions. Reddening or darkening, in itself, can be considered undesirable, because it is a localised effect and accordingly looks different from surrounding skin. Accordingly, users who suffer from localised skin coloration are faced with a dilemma. Do they employ their antiperspirant and locally control perspiration, but suffer from a visible localised darkening or do they avoid using an antiperspirant and suffer from wet patches and risk offending their compatriots by virtue of the body mal-odours? However, such people can face the future and their fellow compatriots with a new confidence because their problem is being addressed.

WO-A-02/102337, EP-A-1428521 and US-A-6086887 each disclose one or more compositions containing an astringent antiperspirant salt and a natural oil comprising a triglyceride of a C18 unsaturated carboxylic acid. However, none contemplate such compositions inhibiting skin darkening arising from application to underarm skin of an antiperspirant composition.

Although EP-A-691125 discloses compositions containing both an α-ω alkane dicarboxylic acid and inter alia a tri-glyceride monocarboxylic acid ester, it does not contemplate the inhibition of skin darkening arising from application to underarm skin of an antiperspirant composition.

It is an object of the present invention to provide a cosmetic method that simultaneously employs an antiperspirant or deodorant effective amount of an astringent aluminium and/or zirconium salt and which ameliorates or overcomes skin darkening or encourages skin lightening.

### Brief summary of the present invention

According to one aspect of the present invention, there is provided a cosmetic method of inhibiting skin darkening according to claim 1.

Herein the term cosmetic method is employed in its conventional manner, that is to say indicates that the method is non-therapeutic.

Percentages of the composition herein are by weight, based on the residue of the composition after the weight of any propellant has been excluded, unless otherwise stated.

By topically applying a combination of constituents in accordance with claim 1 to skin to regions of the body such as the underarm where there is a high density of sweat glands and/or an occluded region where bacteria can otherwise thrive if not subjected to externally applied control, it is possible to achieve the objective of inhibiting sweat production locally at the same time as inhibiting or preventing the skin darkening which can arise when a corresponding antiperspirant composition is topically applied.

Without being bound by any particular theory as to the mechanism by which such a beneficial outcome is achieved, it is believed that the selection of the darkening-inhibiting system described herein, namely the triglyceride oil optionally together with a co-operative active carboxylic acid in a suitable relative concentration to the astringent antiperspirant salt to inhibit skin darkening or to assist in or promote the lightening the colour of the skin relative to that arising from topical contact with an antiperspirant composition, possibly by counteracting skin darkening that is induced by one or more other constituents of the antiperspirant composition and in particular the antiperspirant salt.

The triglyceride oil is capable of being hydrolysed in situ and thereby act as a reservoir of activity over an extended period, as is desirable for a leave on material (the antiperspirant) which remains on the skin until the person next washes the region to which antiperspirant has been applied. That is often at least 4 hours and can conceivably be from 8 to 24 hours. Accordingly, a material which is slowly hydrolysed in situ offers potential benefit. An alternative approach that in theory could be contemplated comprises employing a similar amount of the acids that are hydrolysable from the glycerides. Whilst such acids would be expected to control irritation, the shock application of such a concentration can induce darkening. Thus, proposals for controlling irritation do not inevitably teach control of skin darkening.

It is to be understood that various antiperspirant formulations disclosed in prior published patent (application) specifications have contemplated the incorporation of one or more triglyceride oils, for example as a natural emollient oil. WO-A-02/102337 disclosed antiperspirant compositions that could contain triglyceride oils as precursors for the in-situ generation, by hydrolysis, of PPAR-activators, thereby acting as anti-irritants on the skin, though explicit examples specified the PPAR-activators themselves, not the precursors. EP-A-1428521 disclosed antiperspirant compositions containing a triglyceride oil and glycerol within specified weight ratios, recognising the benefit of such a combination to combat skin damage caused by shaving. US 60806887 discloses antiperspirant compositions containing borage oil. US 5871717 and US 6231842 contemplate the use of triglyceride waxes to gel antiperspirant compositions. However, none of the disclosures in the above-mentioned specifications have taught that if employed at an appropriate ratio to the antiperspirant active, skin darkening, and particularly underarm skin darkening can be inhibited by incorporating the triglyceride oil into a base antiperspirant composition. WO 99/47110 and corresponding US 602296, US 6042841 and US 6365175 contemplate incorporation of petroselinic acid or oils containing it in skin treatment compositions, but such compositions do not contain an astringent antiperspirant active, and accordingly provide no teaching in relation to antiperspirant compositions or amelioration of side-effects that can arise from using antiperspirant compositions.

### Detailed Description of the Invention, including preferred embodiments.

The invention method employed herein topically applies a composition containing at least two essential constituents plus an optional third constituent which act in regard to control sweating/odour generation and the colour of the skin to which the composition is applied.

The deodorant or antiperspirant astringent salt is preferably incorporated in an amount of from 0.5-60% of the composition, excluding the weight of any propellant, the actual concentration present normally being selected in relation to the particular applicator to be employed and whether deodorancy alone is acceptable or antiperspirancy is needed too. For application from contact applicators, the concentration of the astringent salt is often at least 1%, and especially suitably is at least 5% of the composition (which in practice for contact applicators does not contain a propellant), and is desirably less than 30%. In some compositions a preferred concentration of astringent salt is from 10 to 26%. For non-contact applicators some composition do not contain a propellant, and in such compositions, the concentration of such astringent salt is often selected in the range of from 5 to 20%. For compositions containing a propellant, the latter often constitutes from 35 to 90% of the entire composition, that is to say the propellant plus the residue (base composition), and in such aerosol compositions the proportion of the astringent salt in the base composition is often from 10 to 50%, and in many instances from 30 to 50%.

Antiperspirant actives for use herein are selected from aluminium, zirconium and mixed aluminium/zirconium salts, including both inorganic salts, salts with organic anions and complexes. Particularly preferred astringent salts are halohydrate salts, and especially chlorohydrate salts, optionally activated. For aerosol compositions, the antiperspirant active is preferably free from zirconium.

Aluminium halohydrates are usually defined by the general formula Al₂(OH)ₓQ_{y}.wH₂0 in which Q represents chlorine, bromine or iodine, x is variable from 2 to 5 and x + y = 6 while wH₂O represents a variable amount of hydration. Especially effective aluminium halohydrate salts, known as activated aluminium chlorohydrates, are described in EP-A-6739 (Unilever NV et al), the contents of which specification is incorporated herein by reference.

Zirconium astringent salts for employment herein can usually be represented by the empirical general formula: ZrO(OH)_{2n-nz}B_{z}.wH₂0 in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by wH₂0. Preferably, B represents chloride. Preferably, the variable z lies in the range from 1.5 to 1.87. In practice, such zirconium salts are commonly not employed by themselves, but as a component of a combined aluminium and zirconium-based antiperspirant.

The above aluminium and zirconium salts may have co-ordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂(NH₂)COOH. Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis. Alternatively, the complex can be preformed with a polyhydric aliphatic alcohol, such as propylene glycol or glycerol. A complex with a chlorohydrate is commonly referred to as a chlorhydrex.

Mixtures of two or more astringent salts can be employed, but, however, it is particularly preferred to employ astringent salts that are free from zirconium, such as aluminium chlorohydrates and so-called activated aluminium chlorohydrates.

The proportion of solid antiperspirant salt in a suspension composition normally includes the weight of any water of hydration and any complexing agent that may also be present in the solid active. However, when the active salt is incorporated in solution in a hydrophilic solvent such as a glycol, its weight commonly excludes any water present.

If the composition is in the form of an emulsion the antiperspirant active will be dissolved in the hydrophilic phase, which commonly comprises water itself, optionally together with one or more water-miscible liquids. The hydrophilic phase often comprises a disperse phase, though in some instances may alternatively constitute the continuous phase. In emulsions, the antiperspirant active will often provide from 3 to 60% by weight of the hydrophilic phase, particularly from 10% or 20% up to 55% or 60% of that phase.

Alternatively, the composition may take the form of a suspension in which antiperspirant active in particulate form is suspended in a water-immiscible liquid carrier. Such a composition will probably not have any separate aqueous phase present and may conveniently be referred to as "substantially anhydrous" although it should be understood that some water may be present bound to the antiperspirant active or as a small amount of solute within the water-immiscible liquid phase. In such compositions, the particle size of the antiperspirant salts often falls within the range of 0.1 to 200 µm with a mean particle size often from 1 to 20µm.

The particulate antiperspirant active may be present in the form of hollow spheres or dense particles (by which is meant particles which are not hollow) or a mixture of both, at the discretion of the manufacturer.

### Skin darkening-inhibiting system

Herein, the skin darkening-inhibiting system for use in the cosmetic method comprises a triglyceride oil optionally together with an active carboxylic acid. The triglyceride oil is commonly an oil extracted from plants, often a derivative of C₁₈ carboxylic acids, commonly linear, though it can be produced synthetically. Such oils are especially suitably glyceryl esters of one or more unsaturated C₁₈ fatty acids. The fatty acid residues in the oils can comprise, commonly, from one to three olefinic unsaturated bonds and often one or two. Whilst in many instances, the olefinic bonds adopt the trans configuration, in a number of desirable products the bond or bonds adopt the cis configuration. If two or three olefinic unsaturated bonds are present, they can be conjugated. The fatty acid residue can also be substituted by an hydroxyl group. The natural oils employable herein desirably comprise one or more triglycerides of oleic acid, linoleic acid, linolenic acid or ricinoleic acid. Various isomers of such acids often have common names, including linolenelaidic acid, trans 7-octadecenoic acid, parinaric acid, pinolenic acid, punicic acid, petroselenic acid and stearidonic acid. It is especially desirable to employ glycerides derived from oleic acid, linoleic acid, ricinoleic acid or petroselenic acid, or a mixture containing one or more of them.

Natural plant oils containing one or more of such triglycerides include coriander seed oil for derivatives of petroselinic acid, impatiens balsimina seed oil, parinarium laurinarium kernel fat or sabastiana brasilinensis seed oil for derivatives of cis-parinaric acid, dehydrated castor seed oil, for derivatives of conjugated linoleic acids, borage seed oil and evening primrose oil for derivatives of linoleic and linolenic acids, aquilegia vulgaris oil for columbinic acid and sunflower oil or safflower oil for derivatives of oleic acid, often together with linoleic acids. Other suitable oils are oils obtainable from hemp, which can be processed to derive stearadonic acid derivatives, and maize corn oil. An especially convenient natural oil by virtue of its characteristics and availability comprises sunflower oil, ranging from those rich in oleic acid glycerides to those rich in linoleic acid glycerides, rich indicating that its content is higher than that of the other named acid. Other convenient oils include castor oil which comprises esters of ricinoleic acid.

The proportion of the triglyceride oil in the composition is often selected in the range of from 1 to 10% by weight, and especially in the range of from at least 2% by weight. In embodiments in which the triglyceride oil is employed in conjunction with an active acid, at for example from 0.5 to 2%, its concentration is commonly not greater than 6% by weight. In a number of such desirable, the glyceride oil or mixture of oils is employed in a proportion of 2%, 4% or 6% of the total weight of the composition. In various other desirable embodiments, and especially when the active acid is employed at a concentration of below 0.5%, the triglyceride oil is employed at a concentration of at least 4%, such as from 6% to 10%.

The third constituent of the composition and particularly desirable employed in the cosmetic method comprises an active acid, selected from
ai) aliphatic dicarboxylic acids,
aii) unsaturated C₁₈ carboxylic acids and aiii) hydroxybenzoic acids.
Suitable ai) aliphatic dicarboxylic acids are linear. Desirably the dicarboxylic acids contain from 6 to 12 carbon atoms and particularly from 8 to 12. One especially suitable example comprises azelaic acid.

The unsaturated aliphatic C₁₈ carboxylic acids, aii), i.e. olefinically unsaturated, (C₁₈ acids, for short) are often linear. The C₁₈ acids normally comprise from 1 to 4 olefinic groups, of which two or more may be conjugated. An example of mono-unsaturated C18 acids is desirably petroselinic acid. Preferably, the C₁₈ acids comprise at least 2 olefinic groups, such as linoleic acids, linolenic acids and stearidonic acids. The linoleic acid is very desirably a conjugated linoleic acid, sometimes including both a cis and a trans bonding, such as cis-9-trans-11 conjugated linoleic acid and trans-10-cis-12-conjugated linoleic acid, or two trans bondings as in trans-9-trans-11 conjugated linoleic acid.

The hydroxybenzoic acid, aiii), preferably comprises a hydroxyl group in ortho relationship to a carboxylic acid group, including, in particular, salicylic acid.

The active carboxylic acid can comprise an individual member of the aforementioned classes, or can if desired employ a mixture of two or more of them, such as for example a mixture of salicylic acid with a linoleic acid, especially a conjugated linoleic acid, or/and with a linear dicarboxylic acid or a mixture of a linoleic acid with a linear dicarboxylic acid. In such mixtures the relative weight of each acid constituent is from a half to twice that of the or each other constituent.

In some embodiments, it is especially beneficial to employ a mixture of active acids, for example using a combination of a C6-12 dicarboxylic acid (ai) with a C18 unsaturated acid (aii) or an hydroxybenzoic acid (aiii), or a mixture of all three acids, (ai), (aii) and (aiii). Desirable weight ratios of the active acids are:

| | |
|---|---|
| ai:aii | 2:1 to 1:2 |
| ai:aiii | 4:1 to 1:1 |
| ai:aii:aiii | 2:1:0.5 to 1:2:1 |

The total weight % of active acid in the composition, when a mixture of active acids is employed.

The proportion of the active acid constituent or total of the constituents ai to aiii is often selected in the range of from 0.25 to 2% of the composition (excluding any propellant), and is preferably selected in the range of 0.5 to 1.5%, such as from 0.75% to 1.25%. When expressed in relation to the astringent salt, the weight ratio is often selected in the range of from 1:4 to 1:40 active acid:astringent salt, and in a number of highly desirable embodiments is from 1:7.5 to 1:25.

The triglyceride oil conveniently is present in a weight ratio to the astringent salt that takes into account any active carboxylic acid that is additionally present. In the presence of from 0 to 0.5% of such active acid, and especially in contact formulations, the weight of triglyceride is often chosen in the range of from 4 to 10% (%s being by weight of composition excluding any propellant). In a number of other embodiments, and especially contact formulations, the weight of triglyceride is often chosen in the range of from 2 to 6%, together with a proportion of from 0.5 to 2%, particularly up to 1.5%, for example from 0.75 to 1.25% of the active carboxylic acid.

The weight ratio of skin darkening-inhibiting system to the astringent antiperspirant salt, i.e. the ratio x:y is 1:<4 and normally is 1:>1, in which x represents the weight concentration of said triglyceride oil plus 4 times the weight concentration of active carboxylic acid and y represents the weight concentration of said astringent salts. Preferably, the ratio x:y of the darkening-inhibiting system to the astringent salt in the composition is 1:≤3 (i.e. y is equal to or less than 3) and in many desirable embodiments is 2:≤5. The ratio x:y is usually 4:≥5 and in many desirable embodiments is 2:≥3, such as in the region of 2:5 to 3:5. For example, if the antiperspirant salt is present at a concentration of 24%, and the active acid:triglyceride oil weight ratio is 1:4, the minimum triglyceride oil concentration is 3%.

The employment of the system of triglyceride oil, preferably with active carboxylic acid, enables skin darkening to be mitigated for those persons who demonstrate a propensity to be thus affected. This is conveniently described as inhibiting skin darkening, but, by comparison with the skin in the absence of the oil or oil/active acid system, the user can perceive her/his skin to be lighter, so that this could also be regarded as skin lightening.

In addition to the foregoing constituents, compositions employed in the instant invention can contain, if desired, glycerol, for example in a weight ratio to the astringent salt selected in the range of up to 1:2, such as from 1:16 to 2:5. The glycerol can be incorporated as an individual constituent of the composition or may alternatively at least in part be present as an adduct of, or complex with the astringent salt, or absorbed on a particulate constituent. In a number of desirable embodiments, the glycerol is present at a concentration of from 1 to 5% of the composition, and in the same or other embodiments at a weight ratio of from 2:3 to 1:3 to the triglyceride oil.

The composition can contain, if desired, a chelating agent that is capable with complexing with a transition metal ion, such as particularly iron. Such chelating agents are conveniently aminopolycarboxylic acids, such as ethylenediaminetetraacetic acid (EDTA) or pentetic acid (DTPA). The chelating agent is conveniently present at a concentration of up to 1% of the composition, and in many instances 0% or 0.025% up to 0.25% such as from 0.05% to 0.15%.

The compositions that are topically applied in accordance with the instant invention can be in the form of firm solids, soft solids, gels, creams, and liquids and are dispensed using applicators appropriate to the physical characteristics of the composition. The compositions can include additional constituents appropriate for dispensing by such applicators.

Compositions for use in a method according to the present invention often comprise a liquid carrier for the antiperspirant or deodorant salt. Such liquid carrier can be hydrophobic for suspending the salt or hydrophilic for dissolving the salt, or comprise a mixture of both hydrophilic and hydrophobic liquids, the salt typically dissolving in the hydrophilic liquid and one of the two liquids being dispersed in the other to form an emulsion or a microemulsion. The liquid carrier or mixture of carriers often constitutes from 30 to 95% by weight of the composition and in many instances from 40 to 80%.

Hydrophobic liquid carriers commonly can comprise one or more materials having selected within the chemical classes of siloxanes, hydrocarbons, branched aliphatic alcohols, esters and ethers that have a melting point not higher than 25°C and a boiling point of at least 100°C. It will be recognised that the triglyceride oils satisfy such requirements and accordingly contribute to the overall liquid carrier content of the composition.

The siloxanes can be volatile or non-volatile, volatile indicating a measurable vapour pressure at 20 or 25°C. Typically the vapour pressure of a volatile silicone lies in a range from 1 Pa to 2 kPa at 25°C. Volatile siloxanes such as volatile D4-D6 cyclodimethicones or linear dimethicones are especially desirable, and particularly cyclodimethicones comprising D5 (such as DC345) or blends containing at least 80% D5 and/or D6.

Non-volatile silicone oils useful herein usually comprise linear alkylarylpolysiloxanes containing up to 4 or 5 siloxane silicon atoms, such as methylphenylsiloxanes often in which there is from 0.5 to 1.2 phenyl substituent per methyl substituent, as for example in DC704™ available from Dow Corning, Inc. Other non-volatile silicones comprise intermediate and higher molecular weight linear dimethicones that are liquid at 20°C, such as members of the DC200™ series of silicone oils having a viscosity of at least 1 mPa.s, available from Dow Corning, Inc.

Volatile hydrocarbon oils comprise paraffin oils. Non-volatile hydrocarbon oils, which often contain on average between 20 and 40 carbon atoms, include mineral oil and hydrogenated polydecene.

Liquid fatty alcohols are normally branched chain alcohols containing from 12 to 25 carbons and several such desirable alcohols contain from 16 to 20 carbons, including isostearyl alcohol and octyl-decylalcohol.

Liquid fatty alcohol esters include fatty alcohol esters of naphthoic or especially benzoic acid. In such esters the fatty alcohol is often linear, and in many instances contains from 12 to 20 carbon atoms, such as C₁₂-C₁₅, or a mixture of chain lengths. The term also encompasses glyceride oils.

Liquid polyalyleneglycol ethers commonly comprise a polypropyleneglycol polyglycol/polypropylene glycol moiety of from 5 to 20 units terminating in an alkyl ether of from 2 to 6 carbons, such as butyl or t-butyl. A suitable example is obtainable under the CTFA INCI approved name of PPG-14-butyl ether.

Hydrophilic carrier liquids that can be employed in compositions herein commonly comprise water and/or a mono or polyhydric alcohol or water-miscible homologue. Monohydric alcohols often are short chain, by which is meant that they contain up to 6 carbons, and in practice are most often ethanol or sometimes iso-propanol. Polyhydric alcohols commonly comprise ethylene or propylene glycol, or a homologue can be employed such as diethylene glycol.

There are broadly speaking two classes of carrier materials that are employed herein, hydrophobic viz water-immiscible liquids forming one class and hydrophilic such as water and dihydric alcohol forming a second.

Both hydrophobic and hydrophilic carriers can be employed in the same composition, commonly in a weight ratio of from 100:1 to 1:100. To reduce the likelihood of the liquids separating into distinct layers, it is conventional for an emulsifier or mixture of emulsifiers to be employed, thereby encouraging one phase to be retained as a dispersion within the other, continuous phase. The proportion of emulsifier in antiperspirant emulsions is normally selected in the range of from 0.1 to 8% by weight, and in many embodiments is from 0.5 to 5%. Suitable emulsifiers commonly have an HLB value in the region of from 2 to 10 and often in the region of 3 to 8. In a mixture of emulsifiers, all may have an HLB value in the aforementioned regions or one or more may have a higher HLB value, such as from 10 to 16, provided that the weight averaged HLB value is up to 10 or particularly up to 8. Classes of emulsifiers commonly comprise nonionic surfactants having such an HLB value, including polyalkylene oxide esters or ethers, such as polyethylene oxide (POE) and/or poly propylene oxide (POP) esters or ethers optionally containing a glyceryl unit and/or fatty ester or ether derivatives of a polyhydroxyaliphatic or cycloaliphatic group containing from 3 to 6 carbons, such as glycerol or sorbitol. The number of POE and/or POP units in nonionic surfactant emulsifiers is commonly between 2 and 100 and particularly on weight average between 3 and 25 units and in many instances on average between 4 and 10.

In many non-ionic surfactants desirable as emulsifiers herein, the hydrophobic component therein is usually provided by the alkyl residue of a fatty alcohol or acid, in many instances containing from 12 to 30 carbons, and in particular one or more palmityl, cetyl stearyl and/or eicosonyl or behenyl groups. Of these, stearyl and a mixture of stearyl and cetyl are especially favoured.

Another suitable class of emulsifiers, particular interesting when the formulation comprises a significant fraction of a silicone oil, comprises alkyl dimethicone copolymers, namely polyoxyalkylene modified dimethylpolysiloxanes. The polyoxyalkylene group is often a POE or POP or a copolymer of POE and POP. The copolymers often terminate in C₁ to C₂₂ alkyl groups, particularly C₁₂ to C₁₈. Suitable examples comprise cetyl dimethicone copolymers available as Abil EM90™ or EM97™ from Th.Goldschmidt.

The compositions that remain in liquid form can be applied employing conventional applicators such as a roll-on or by being pumped or squeezed through a spray-generating orifice. Such compositions may be thickened, for example using one or more thickeners described subsequently herein, though usually their viscosity, as measured at 25°C by a Brookfield viscometer fitted with a Tbar rotating at 20rpm, is not more than 1000mPa.s.

The second class can be thickened by water-soluble or dispersible materials of higher viscosity, including various of the emulsifiers, and/or thickened or gelled with water-soluble or water-dispersible polymers including polyacrylates, and water-soluble or dispersible natural polymers, such as water-soluble polysaccharide or starch derivatives, such as alginates, caragheenan, agarose and water-dispersible polymers include cellulose derivatives. An aqueous phase can also be thickened in accordance with known technology using a dispersion of a water-insoluble particulate material, such a finely divided clay, possibly in conjunction with an electrolyte or polyelectrolyte including a water-soluble emulsifier.

Dihydric alcohols, optionally containing ether links such as those described above also can be gelled using dibenzylidene alditols, such as for example dibenzylidene sorbitol. Water-immiscible carrier fluids, such as those described hereinabove, can be thickened or structured using a wide range of thickeners, and oil-soluble gellants and structurants that are known to the skilled producer. Thickeners for such carrier liquids include particulate inorganic substances which are sometimes alternatively referred to as suspending agents particularly if the eventual use of the formulation is in an aerosol, such as clays or finely divided silica. Such thickeners are well suited to increasing the viscosity for liquids, but can also produce semi-solids (soft solids) provided that sufficient thickener is employed.

Other materials which can act as thickeners for water-immiscible liquids, but many of which can also act as gellants or structurants by increasing their concentration in the liquid, can comprise organic polymers which are soluble in the carrier liquid(s), though commonly at elevated temperature of above 60°C. Such polymers are particularly well suited to producing compositions in the form of soft or firm solids.

Such polymers can be selected from polysaccharides esterified with a fatty acid of which one excellent example comprises dextrin palmitate: polyamides as discussed in US 5500209, such as the product available under the trade name Versamid 950™ that is derived from hexamethylene diamine and adipic acid; alkylene/arylene block copolymers, for example styrene and ethylene, propylene and/or butylene block copolymers eg SEBS block copolymers, many of which are available under the trade name Kraton™; alkyl substituted galactomannan such as N-HANCE™: co-polymers of vinyl pyrrolidone with polyethylene containing at least 25 methylene units. The concentration of such polymers in the water-immiscible liquid is often selected in the range of from 1 to 20%, depending on the extent of thickening or structuring required, and the effectiveness of the chosen polymer in the liquid/mixture.

One class of structurant which is desirable by virtue of its long standing proven capability to produce firm solids and more recently in making soft solids, comprises waxes. Herein, the term wax is employed to encompass not only materials of natural origin that are solid with a waxy feel and water-insoluble at 30-40°C, but melt at a somewhat higher temperature, typically between 50 and 95°C, such as beeswax, candelilla or carnauba wax, but also materials having similar properties. Such other waxes include hydrocarbon waxes, eg paraffin wax, mineral wax and microcrystalline wax; synthetic waxes, such as polyethylene of 2000 to 10000 daltons; waxy derivatives or waxy components of natural waxes, such as ester components, either extracted or synthesised, solid ester derivatives of glyceryl or glycol, typically with linear saturated fatty acids, usually containing a significant fraction of C₁₆₋₂₂ acid residues, which may be synthesised or obtained by hydrogenating the corresponding natural oil; petroleum waxes, waxy silicone polymers containing alkyl substituents of at least C₁₀ chain length; and, importantly, waxy fatty alcohols, that normally are linear and often contain from 14 to 24 carbons, such as stearyl alcohol, cetyl alcohol and/or behenyl alcohol.

Further classes of structurants for water-immiscible liquids that are employable herein, in accordance with their disclosure in patent literature relating to the preparation of antiperspirant formulations in soft solid or firm stick form include oil-soluble polyamides or amide/silicone copolymers, hydroxystearic acid, such as 12-hydroxystearic acid, or ester or amide derivatives thereof, N-acyl amino acid amides and esters described in US-A-3969087, such as, in particular, N-Lauroyl-L-glutamic acid di-n-butylamide; amide derivatives as set forth in WO 98/27954 notably alkyl N,N'dialkyl succinamides; threitol or like amido gellants as set forth in US-A-6410001; lanosterol, as set forth in US-A-6251377; amido derivatives of cyclohexane as set forth in US-A-6410003;a combination of a sterol and a sterol ester as set forth in WO 00/61096, eg γ-oryzanol and β-sitosterol; and fatty acid esters of cellobiose, such as in particular a product containing predominantly cellobiose octanonanoate and a minor fraction of cellobiose heptanonanoate.

Mixtures of materials within each class of gellant/structurant can be employed.

Compositions that are firm solids, commonly obtained by use of a gellant or structurant, can be applied employing a stick applicator and soft solids, gels and creams can be applied employing an applicator having a dispensing head provided with at least one aperture through which the soft solid, gel or cream can be extruded under mild pressure.

When the antiperspirant composition employed herein comprises an aerosol composition, it contains a propellant in addition to a base composition as described herein above, commonly in a weight ratio of from 95:5 to 40:60, and in many formulations, the weight ratio is from 90:10 to 50:50. For the avoidance of doubt, the concentrations of the ingredients in aerosol compositions herein, except for the propellant, are based on the base composition. So, for example, an active carboxylic concentration of 1% in the base compositions corresponds to a concentration of 0.25% in the full aerosol composition when the proportions are 25% base composition and 75% propellant.

The propellant is conveniently a low boiling point material, typically boiling below -5°C, for example an alkane such as propane, butane or isobutane, and possibly containing a fraction of pentane or isopentane, or a hydrofluorocarbon or fluorocarbon of similar carbon content. During filling of the aerosol canister, the propellant gas is liquified by virtue of the elevated pressure that is generated therein. As the skilled man recognises, the base composition for an aerosol composition is typically in the form of a liquid which may have been thickened, but is not gelled or solidified.
The compositions contemplated herein for inhibiting skin darkening can additionally comprise one or more optional constituents which have hitherto been incorporated or proposed for incorporation in antiperspirant compositions. Such optional constituents may be liquid or solid, and normally comprise in total not more than 10% by weight of the composition. Such optional constituents can comprise sensory modifiers, such as talc or finely divided polyethylene, such as in an amount of up to 5% by weight; fragrance, including, if desired deoperfumes, often in an amount of up to 4%, eg 0.3 to 2% by weight, colourants; skin cooling agents such as menthol; wash-off agents such as non-ionic surfactants.

The compositions contemplated herein for inhibiting skin darkening can be made by the skilled man using methods known in the antiperspirant industry or described in published literature for the preparation of antiperspirant roll-on, squeeze or pump spray cream or soft solid or firm stick compositions.

In some preferred compositions, the C₁₈ triglyceride oil is complemented by an active acid as described herein and particularly in ratios of triglyceride oil to active acid described hereinbefore. One particularly desirable combination comprises the C₆₋₁₂ aliphatic dicarboxylic acid such as azelaic acid (which is especially desirable) or the hydroxybenzoic acid such as salicylic acid with the triglyceride oil. A further combination, which is advantageous, and is particularly desirable if for some reason azelaic acid is not employed, comprises the triglyceride oil and a linoleic acid, such as a conjugated linoleic acid. In certain especially desirable embodiments, the triglyceride oil is at least partly derived from the same unsaturated C₁₈ acid as is the active acid that is employed in conjunction with the oil.

The compositions employed in the instant invention can be made is a conventional manner and in conventional equipment known by the skilled man to be appropriate for the physical type of composition he wishes to make.

By way of example, a liquid composition is conventionally made by mixing the ingredients together in a vat, usually at a temperature conducive to dissolution or above the melting point of ingredients, and possibly with pre-mixing of the antiperspirant active with a solvent to ensure optimum dissolution. Emulsions often contain an intermediate heating step to above the melting point of the emulsifiers, such as from 50 to 70°C. If the liquid composition comprises two liquid phases, the composition is often subjected to shear mixing to encourage emulsion formation. Compositions comprising a particulate antiperspirant suspended in a thickened carrier liquid can be formed at a temperature at which the thickener is dispersed throughout or dissolved in the carrier liquid, which can range from ambient through to an elevated temperature, possibly up to 60 or 70°C, depending on the choice of thickener/carrier. If the composition is in the form of a soft solid or firm stick, this is commonly obtained by incorporating a gellant, or structurant into a carrier liquid and the gellant is often premixed with the carrier liquid (or a fraction of it) at an elevated temperature, eg at above 70-80°C, until dissolution occurs. The remaining ingredients are then progressively added whilst the composition remains above its setting temperature, the composition is introduced into a mould or dispensing container and finally cooled or permitted to cool to ambient. Creams employing a thickener are often prepared in a similar manner to liquids, though if they include an organic gellant, a similar process to that for sticks may be employed. Aerosol formulations commonly comprise first the formation of a liquid formulation which is introduced into a aerosol container, the valve is fitted and propellant forced under pressure into the container. Simultaneous inhibition of skin darkening and inhibition of sweating according to a further aspect of the present invention is carried out by topically applying to human skin a composition containing an antiperspirant or deodorant composition as described hereinafter in accordance with claim 1 in a sufficient amount to enable the astringent salt, eg Al or Al/Zr salt contained in the composition to act as an antiperspirant. Normally, an antiperspirant effect is observable when the astringent salt is applied at a density of at least 2g/m², and many users apply from 4 to 6g/m² on average. For the purposes of this invention, such density can actually measured or calculated by measuring the area of skin to which composition is applied, and the weight loss of composition from the applicator, knowing the concentration of astringent salt in the composition. It will be recognised that the actual amount of composition that should be deposited will vary with the applicator type, indeed each user tends to regulate the amount used, but commonly for many users the weight of composition (excluding propellant) deposited falls within the range of 5g/m² to 35g/m².

Having provided a detailed description of the invention, it will now be illustrated by the following Examples.

Ingredients employed in the Examples are as follows:

| | |
|---|---|
| 1 | demineralised water - laboratory production |
| 2 | Aluminium Chlorohydrate, 50% w/w solution Chlorhydrol™ (Reheis) |
| 3 | Cropure™ sunflower seed oil (Croda) |
| 4 | (Seatons) |
| 5 | Volpo S2™ (Croda) |
| 6 | Brij 78™ (Uniqema) |
| 7 | same fragrance throughout |
| 8 | Emerox 1110™ (Cognis) |
| 9 | Conjugated Linoleic Acid (80% active) - Clarinol A80™ |
| 10 | (Clariant) |
| 11 | Di Na salt Nervanaid Ba2™ |
| 12 | Pricerine 9091™ (Uniqema) |
| 13 | Ionol™ (Shell) |
| 14 | 40% aqueous solution Rezal 67™ (Reheis) |
| 15 | Cyclomethicone (mainly D5) - DC245™ (Dow Corning) |
| 16 | Emulgade SE (Cognis) |
| 17 | Solonace (National Starch) |
| 18 | Emulgin B2 (Cognis) |
| 19 | Polawax GP200™ (Croda) |
| 20 | Eumulgin B-2™ (Cognis) |
| 21 | Eutanol G™ (Cognis) |
| 22 | Aluminium Zirconium Chlorohydrate Glycine complex Reach 908™ with adducted 2% glycerol, in house preparation according to the method of WO03/70210 |
| 23 | Activated Aluminium Chlorohydrate A296™ (BK Giulini) |
| 24 | Dimethicone, 10 mPa.s, DC200™ (Dow Corning Inc) |
| 25 | Fluid AP™ (Amerchol) |
| 26 | Finsolv TN™ (Finetex) |
| 27 | Lanette 18™ (Cognis) |
| 28 | Acumist B18™ (Allied Signal) |
| 29 | Castorwax MP80™ (CasChem) |
| 30 | Ultra Talc 3000™ (Ultra Chemical) |
| 31 | Prifac 8961™ (Uniqema) |
| 32 | Brij 700™ (Uniqema) |
| 33 | A296™ (BK Giulini) adducted with 2% glycerol, in house preparation according to the method of WO03/70210 |
| 34 | Bentone 38™ * (Rheox) |
| 35 | DC1501™ (Dow Corning) |
| 36 | Lannette 16™ (Cognis) |
| 37 | fumed silica Aerosil 200™ (Degussa0 |
| 38 | (Fluka) |
| 39 | Aluminium Zirconium Chlorohydrate Glycine complex Reach 908™ (Reheis) |
| 40 | (Fluka) |
| 41 | Disorbene™ (Roquette) |
| 42 | Propanolamine (BASF) |

The weight %s listed in subsequent Tables relate to the ingredient itself and not to the active constituent therein, which may be less than 100% of the ingredient.

### Examples 1-10 and Comparison CA

Representative liquid compositions as summarised in Table 1 below that are capable of being dispensed by a roll-on according to EP1175165 are made by blending the constituents in a conventional manner at a temperature reaching about 65 to 70°C for emulsifier dissolution in a vat, adding the fragrance at about 50-55°C and then pouring them into the dispensing applicator.

Similar formulations can be made employing an aluminiumzirconium chorohydrate complex with glycine (²²) at a concentration of 12.5% instead of the aluminium chlorohydrate (²). Such compositions contain the triglyceride oil at a proportionately higher weight ratio (by a factor of 1.2) that the ratios listed above, and concomitant reduction in water(¹) concentration.

**Table 1**

| Examples | 1 | 2 | 3 | 4 | 5 | 6 | 7 | 8 | 9 | 10 | CA |
|---|---|---|---|---|---|---|---|---|---|---|---|
| Ingredient | % by weight | | | | | | | | | | |
| Water¹ | balance | | | | | | | | | | |
| ACH² | 15 | 15 | 15 | 15 | 15 | 15 | 15 | 15 | 15 | 15 | 17.5 |
| Sunflower Oil³ | 4 | 4 | 4 | 4 | 4 | 4 | 2 | 4 | 4 | 8 | |
| Castor Oil⁴ | | | | | | | 2 | | | | |
| Steareth-2⁵ | 1.92 | 1.92 | 1.92 | 1.92 | 1.92 | 1.92 | 1.92 | 1.92 | 1.92 | 3.84 | 2.6 |
| Steareth-20⁶ | 1.28 | 1.28 | 1.28 | 1.28 | 1.28 | 1.28 | 1.28 | 1.28 | 1.28 | 2.56 | 0.6 |
| Fragrance⁷ | 1 | 1 | 1 | 1 | 1 | 1 | 1 | 1 | 1 | 1 | 1 |
| Azelaic Acid⁸ | 1 | 0.5 | | | | | 0.75 | 1 | 0.6 | | |
| CLA⁹ | | | 1 | 0.5 | | | 0.75 | | 0.6 | | |
| Salicylic acid¹⁰ | | | | | 1 | 0.5 | | 0.5 | 0.3 | | |
| EDTA¹¹ | | 0.1 | 0.1 | | 0.1 | | | 0.1 | 0.1 | 0.1 | |
| Glycerol¹² | | | | | | | | | | 4 | |
| BHT¹³ | 0.05 | 0.05 | 0.05 | 0.05 | 0.05 | 0.05 | 0.05 | 0.05 | 0.05 | 0.05 | 0.05 |

The formulations exhibit antiperspirancy as shown by a conventional test in which the composition is applied to one underarm (L/R randomised) of a panel of persons aged between 18 and 55 and the weight of sweat generated under standard test conditions for a standard period of time as measured and compared with the weight of sweat generated in the other underarm, to which the composition is not applied.

The darkening of underarm skin by application of the invention and standard compositions is assessed by a test in which the composition is applied using a roll-ball applicator according to EP1175165, typically applying from 0.2 to 0.3g composition per armpit at each application.. Product is applied over a four week period (with four product applications per product per day), to give skin darkening an opportunity to develop. The test product is applied to one armpit of a panel of ca. 30 testers and a standard antiperspirant composition is applied to their other armpit (L/R randomised). The panel is selected from persons who do not exhibit significant underarm darkening at the start of the test. During the 4-week test underarm skin colour is regularly assessed by a skilled and experienced assessor by comparing the darkest part of each armpit with the tester's base skin colour which is taken to be the skin colour of the adjacent upper inner arm. This data is averaged over the panel of testers and a mean hyper-pigmentation score is obtained for each sample (be it invention or standard) composition. The scores for the invention composition samples are then compared with the standard composition and each is seen to exhibit a lower score than that for the standard composition confirming that the invention compositions inhibit skin darkening.

### Examples 11 and 12 and Comparison CB

Representative pump spray compositions summarised in Table 2 below are made by a conventional method and filled into conventional pump spray dispensers prior to application and on testing in the manner of Examples 1-10 show inhibition of skin darkening.

**Table 2**

| Example | 11 | 12 | CB |
|---|---|---|---|
| Constituent | % by weight | | |
| Al-Zr Pentachloro-hydrate¹⁴ | 50.0 | 50.0 | 50.0 |
| Waster¹ | balance | | |
| Cyclomethicone¹⁵ | 2.0 | 4.0 | 10.0 |
| Glycerol¹² | 4.0 | 2.0 | 2.0 |
| Sunflower Oil³ | 8.0 | 5.0 | |
| Azelaic Acid⁸ | | 1.0 | |
| Glyceryl Stearate, Ceteareth-20, Ceteareth-12, Cetearyl Alcohol, Cetyl Palmitate¹⁶ | 2.0 | 2.0 | 2.0 |
| Amphoteric Potato Starch¹⁷ | 1.0 | 1.0 | 1.0 |
| Perfume | 1.0 | 1.0 | 1.0 |
| Glyceryl stearate¹⁸ | 1.0 | 1.0 | 1.0 |
| Cetearyl Alcohol, PEG 20 Stearate¹⁹ | 0.65 | 0.65 | 0.65 |
| Ceteareth 20²⁰ | 0.4 | 0.4 | 0.4 |
| Octyldodecanol²¹ | 0.5 | 0.5 | 0.5 |

### Examples 13 to 17 and Comparison CC

Representative stick compositions summarised in Table 3 below are made by a conventional method and filled into firm stick dispensers as described in US-A-6598767 prior to application and on testing in the manner of Examples 1-10 show inhibition of skin darkening.

**Table 3**

| Examples | 13 | 14 | 15 | 16 | 17 | CC |
|---|---|---|---|---|---|---|
| Constituent | % by weight | | | | | |
| Cyclomethicone¹⁵ | Balance | | | | | |
| AZAG*²² | 24.0 | | 24.0 | | 24.0 | |
| AACH²³ | | 24.0 | | 24.0 | | 24.0 |
| non-volatile silicone²⁴ | 7.5 | | 7.5 | 7.5 | 7.5 | 7.5 |
| PPG-14 butyl ether²⁵ | 6.0 | | 6.0 | | | 6.0 |
| C₁₂₋₁₅ alkyl benzoate²⁶ | | 6.0 | | 6.0 | 6.0 | |
| Stearyl alcohol²⁷ | 14.5 | 14.5 | 14.5 | 14.5 | 14.5 | 14.5 |
| polyethylene powder²⁸ | 3.0 | 3.0 | 3.0 | 3.0 | 3.0 | 14.5 |
| Castor wax²⁹ | 2.0 | 2.0 | 2.0 | 2.0 | 2.0 | 2.0 |
| Talc³⁰ | 2.0 | 2.0 | 2.0 | 2.0 | 2.0 | 2.0 |
| Glycerol* | 2.0 | | 2.0 | | 2.0 | |
| Sunflower oil³ | 8.0 | 5.0 | 5.0 | 5.0 | 5.0 | |
| Azelaic Acid⁸ | | 1.0 | | | | |
| CLA⁹ | | | 1.0 | | | |
| Salicylic acid | | | | 1.0 | | |
| Sunflower Oil acids³¹ | | | | | 1.0 | |
| Fragrance | 1.0 | 1.0 | 1.0 | 1.0 | 1.0 | 1.0 |
| Steareth 100³² | 0.7 | 0.7 | 0.7 | 0.7 | 0.7 | 0.7 |

| | | | | | | |
|---|---|---|---|---|---|---|
| Ingredients marked * in these Examples were combined in a prior preparative stage before the remainder of the constituents were brought together. | | | | | | |

### Examples 18 to 20 and Comparison CD

Representative aerosol formulations summarised in Table 4 below are made by a conventional method and filled into aerosol dispensers as described in EP1044055B prior to application, pressurised with propellant and on testing in the manner of Examples 1-10 show inhibition of skin darkening.

**Table 4**

| Examples | 18 | 19 | 20 | CD |
|---|---|---|---|---|
| Base composition | % by weight | | | |
| AACH*³³ | 39.0 | 39.0 | 39.0 | 39.0 |
| cyclomethicone¹⁵ | 18.8 | 19.8 | 20.2 | 23.0 |
| PPG-14 butyl ether²⁵ | 21.8 | 22.8 | 23.4 | 26.6 |
| Fragrance | 5.4 | 5.4 | 5.4 | 5.4 |
| Hydrophobic clay³⁴ | 4.2 | 4.2 | 4.2 | 4.2 |
| Sunflower oil³ | 7.0 | 5.0 | 3.0 | |
| Glycerol* | 2.0 | | | |
| Castor oil⁴ | | | 2.0 | |
| Azelaic Acid⁸ | | 1.0 | | |
| CLA⁹ | | | 1.0 | |
| Octyldocecano1²¹ | 1.0 | 1.0 | 1.0 | 1.0 |
| Silicone Gum³⁵ | 0.8 | 0.8 | 0.8 | 0.8 |

To make the pressurised aerosol composition, 1 part by weight of each base composition was introduced into an aerosol canister followed by 3 parts by weight of a propellant comprising a mixture of propane, butane and isobutene CAP40™³⁷.

### Example 21 to 23 and Comparison CE

Representative Soft Solid Formulations summarised in Table 5 below are made by a conventional method and filled into conventional soft stick dispensers prior to application and on testing in the manner of Examples 1-10 show inhibition of skin darkening.

**Table 5**

| Examples | 21 | 22 | 23 | CE |
|---|---|---|---|---|
| Constituent | % by weight | | | |
| cyclomethicone¹⁵ | 36.7 | 38.8 | 38.8 | 43.3 |
| Particulate AACH*³³ | 24.5 | 24.5 | 24.5 | 24.5 |
| PPG-14 butyl ether²⁵ | 9.5 | 10.4 | 10.4 | 11.9 |
| Castor wax²⁹ | 6.0 | 6.0 | 6.0 | 6.0 |
| cetyl alcohol³⁶ | 6.0 | 6.0 | 6.0 | 6.0 |
| Talc³⁰ | 6.0 | 6.0 | 6.0 | 6.0 |
| Sunflower oil³ | 7.0 | 5.0 | 5.0 | |
| Glycerol* | 2.0 | | | |
| CLA⁹ | | 1.0 | | |
| Salicylic acid¹⁰ | | | 1.0 | |
| Silica³⁷ | 1.5 | 1.5 | 1.5 | 1.5 |
| Fragrance | 0.8 | 0.8 | 0.8 | 0.8 |

### Example 24 to 26 and Comparison CF

Representative gel compositions summarised in Table 6 below are made by a conventional method and filled into conventional gel dispensers prior to application and on testing in the manner of Examples 1-10 show inhibition of skin darkening.

**Table 6**

| Examples | 24 | 25 | 26 | CF |
|---|---|---|---|---|
| Constituent | % by weight | | | |
| Propylene glycol³⁸ | 44.5 | 47.5 | 47.5 | 53.3 |
| AZAG³⁹ | 20.0 | 20.0 | 20.0 | 20.0 |
| Dipropylene glycol⁴⁰ | 11.0 | 11.5 | 11.5 | 12.1 |
| Isostearyl alcohol²¹ | 11.0 | 11.5 | 11.5 | 12.1 |
| Dibenzoyl sorbitol⁴¹ | 3.0 | 3.0 | 3.0 | 3.0 |
| Glycerol¹² | 2.0 | | | |
| Sunflower oil³ | 8.0 | 5.0 | 5.0 | |
| Azelaic Acid⁸ | | 1.0 | | |
| Conjugated Linoleic Acid⁹ | | | 1.0 | |
| 3-amino-1-propanol⁴² | 0.5 | 0.5 | 0.5 | 0.5 |

The principle of inhibiting skin darkening can be further demonstrated by comparative trials in which skin on a panel of volunteers aged 18 to 55 is tanned by a course of controlled UV irradiation with a sun lamp and thereafter side-by side strips of skin are treated twice daily with an antiperspirant composition free from or containing a darkening inhibition system or with no treatment, and the colour of the skin observed for a period of up to 4 weeks and assessed by a skilled assessor against a standard colour chart, the higher the number, the more intense the colour. From those observations mean colour change for the panellists is calculated relative to no treatment. Such tests have indicated the inhibition of darkening with (³):(²) x:y ratio of 8:15, and (³) + (⁸) : (²) at x:y ratio of 8:15 and (³)+(¹⁰): (²) at x:y ratio of 2: 5.

## Claims

1. A cosmetic method of inhibiting or preventing skin darkening arising from topically applying to underarm skin an antiperspirant composition comprising an antiperspirant active salt selected from astringent aluminium and/or zirconium salts by incorporating into the composition a darkening inhibition system comprising a C₁₈ unsaturated carboxylic acid triglyceride oil optionally together with an active carboxylic acid selected from
ai) linear aliphatic dioic acids containing from 6 to 12 carbon atoms;
aii) unsaturated aliphatic C₁₈ carboxylic acids and
aiii) hydroxybenzoic acids,
at a ratio to said astringent salts, x:y, of 1:<4 in which x represents the weight concentration of said triglyceride oil plus 4 times the weight concentration of active carboxylic acid and y represents the weight concentration of said astringent salts.

2. A method according to claim 1 in which the weight ratio x:y is 1:≤3.

3. A method according to claim 1 in which the weight ratio x:y is 2:≤5.

4. A method according to any preceding claim in which the weight ratio x:y is 2:≥3.

5. A method according to any preceding claim in which the weight ratio of triglyceride oil to active carboxylic acid in the composition is selected in the range of up to 8:1 and is preferably at least 2:1.

6. A method according to any preceding claim in which the triglyceride oil comprises sunflower oil, castor oil or a mixture thereof.

7. A method according to any preceding claim in which the linear aliphatic dicarboxylic acid is azelaic acid.

8. A method according to any of claims 1 to 5 in which the unsaturated aliphatic C₁₈ carboxylic acid is a linoleic acid.

9. A method according to claim 8 in which the linoleic acid is a conjugated linoleic acid.

10. A method according to any of claims 1 to 5 in which the hydroxybenzoic acid is salicylic acid.

11. A method according to any preceding claim in which the compositions contains from 1 to 10% by weight of the triglyceride oil.

12. A method according to any preceding claim in which the composition additionally comprises a chelating agent for a transition metal.

13. A method according to claim 12 in which the chelating agent is an aminopolycarboxylic acid.

14. A method according to claim 13 in which the aminopolycarboxylic acid is ethylenediaminetetraacetic acid or diethylenetriaminepentaacetic acid.

15. A method according to any of claims 12 to 14 in which the chelating agent is present in a weight ratio to the hydroxybenzoic acid of from 1:20 to 1:5.

16. A method according to any preceding claim in which the composition additionally comprises glycerol.

17. A method according to claim 16 in which the glycerol is present in the composition at a weight ratio to the triglyceride oil of from 1:1 to 1:4.

18. A method according to any preceding claim in which the composition contains from 4 to 10% by weight of the triglyceride oil and from 0 to less than 0.5% of the active carboxylic acid.

19. A method according to any preceding claim in which the composition contains from 2 to 6% by weight of the triglyceride oil and from 0.5 to 2.0% by weight of the active carboxylic acid.

20. A method according to any preceding claim which the antiperspirant or deodorant active salt comprises an astringent aluminium salt.

21. A method according to claim 19 in which the astringent aluminium salt is an aluminium chlorohydrate.

22. A method according to any preceding claim in which the antiperspirant or deodorant active salt is present at a concentration of from 10 to 50% by weight of the composition excluding any propellant.

23. A method according to claim 21 in which the composition is free from propellant and contains from 10 to 26% by weight of the antiperspirant or deodorant astringent salt.

24. A method according to claim 21 in which the composition contains propellant and from 20 to 45% by weight of the antiperspirant or deodorant astringent salt, %s being based on the composition excluding the propellant.

25. A cosmetic method in accordance with any preceding claim for simultaneously inhibiting sweating and inhibiting skin darkening comprising topically applying to an armpit the antiperspirant composition in an amount sufficient to deposit the astringent antiperspirant salt at a density of at least 2g/m².

## Patentansprüche

1. Kosmetisches Verfahren zur Inhibierung oder Verhinderung eines Dunkelwerdens der Haut, das aus einer topischen Auftragung einer Antiperspirant-Zusammensetzung, die ein Antiperspirant-aktives Salz, ausgewählt aus adstringierenden Aluminium- und/oder Zirkoniumsalzen, umfasst, in der Achselhöhle resultiert, durch Einarbeiten eines Inhibierungssystems für das Dunkelwerden, das ein C₁₈-ungesättigte Carbonsäure-Triglyceridöl gegebenenfalls zusammen mit einer aktiven Carbonsäure, ausgewählt aus
ai) linearen aliphatischen Disäuren, die 6 bis 12 Kohlenstoffatome enthalten;
aii) ungesättigten aliphatischen C₁₈-Carbonsäuren und
aiii) Hydroxybenzoesäuren,
umfasst, in einem Verhältnis zu den adstringierenden Salzen, x:y, von 1:<4, wobei x das Gewichtsverhältnis des Triglyceridöl plus viermal die Gewichtskonzentration an aktiver Carbonsäure darstellt und y die Gewichtskonzentration der adstringierenden Salze darstellt.

2. Verfahren nach Anspruch 1, wobei das Gewichtsverhältnis x:y 1:<3 ist.

3. Verfahren nach Anspruch 1, wobei das Gewichtsverhältnis x:y 2:≤5 ist.

4. Verfahren nach einem vorangehenden Anspruch, wobei das Gewichtsverhältnis x:y 2:≥3 ist.

5. Verfahren nach einem vorangehenden Anspruch, wobei das Gewichtsverhältnis von Triglyceridöl zu aktiver Carbonsäure in der Zusammensetzung in dem Bereich von bis zu 8:1 ausgewählt wird und vorzugsweise wenigstens 2:1 ist.

6. Verfahren nach einem vorangehenden Anspruch, wobei das Triglyceridöl Sonnenblumenöl, Rizinusöl oder ein Gemisch davon umfasst.

7. Verfahren nach einem vorangehenden Anspruch, wobei die lineare aliphatische Dicarbonsäure Azelainsäure ist.

8. Verfahren nach einem der Ansprüche 1 bis 5, wobei die ungesättigte aliphatische C₁₈-Carbonsäure eine Linolsäure ist.

9. Verfahren nach Anspruch 8, wobei die Linolsäure eine konjugierte Linolsäure ist.

10. Verfahren nach einem der Ansprüche 1 bis 5, wobei die Hydroxybenzoesäure Salicylsäure ist.

11. Verfahren nach einem vorangehenden Anspruch, wobei die Zusammensetzungen 1 bis 10 Gewichts-% des Triglyceridöls enthalten.

12. Verfahren nach einem vorangehenden Anspruch, wobei die Zusammensetzung zusätzlich einen Chelatbildner für ein Übergangsmetall umfasst.

13. Verfahren nach Anspruch 12, wobei der Chelatbildner eine Aminopolycarbonsäure ist.

14. Verfahren nach Anspruch 13, wobei die Aminopolycarbonsäure Ethylendiamintetraessigsäure oder Diethylentriaminpentaessigsäure ist.

15. Verfahren nach einem der Ansprüche 12 bis 14, wobei der Chelatbildner in einem Gewichtsverhältnis zu der Hydroxybenzoesäure von 1:20 bis 1:5 vorliegt.

16. Verfahren nach einem vorangehenden Anspruch, wobei die Zusammensetzung zusätzlich Glycerin umfasst.

17. Verfahren nach Anspruch 16, wobei das Glycerin in der Zusammensetzung in einem Gewichtsverhältnis zu dem Triglyceridöl von 1:1 bis 1:4 vorliegt.

18. Verfahren nach einem vorangehenden Anspruch, wobei die Zusammensetzung 4 bis 10 Gewichts-% des Triglyceridöls und 0 bis weniger als 0,5 % der aktiven Carbonsäure enthält.

19. Verfahren nach einem vorangehenden Anspruch, wobei die Zusammensetzung 2 bis 6 Gewichts-% des Triglyceridöls und 0,5 bis 2,0 Gewichts-% der aktiven Carbonsäure enthält.

20. Verfahren nach einem vorangehenden Anspruch, wobei das als Antiperspirant oder Deodorant aktive Salz ein adstringierendes Aluminiumsalz umfasst.

21. Verfahren nach Anspruch 19, wobei das adstringierende Aluminiumsalz ein Aluminiumchlorhydrat ist.

22. Verfahren nach einem vorangehenden Anspruch, wobei das als Antiperspirant oder Deodorant aktive Salz in einer Konzentration von 10 bis 50 Gewichts-% der Zusammensetzung, ausgenommen ein Treibmittel, vorliegt.

23. Verfahren nach Anspruch 21, wobei die Zusammensetzung frei von Treibmittel ist und 10 bis 26 Gewichts-% des adstringierenden Antiperspirant- oder Deodorant-Salzes enthält.

24. Verfahren nach Anspruch 21, wobei die Zusammensetzung Treibmittel und 20 bis 45 Gewichts-% des adstringierenden Antiperspirant- oder Deodorant-Salzes enthält, wobei Prozentangaben auf die Zusammensetzung, ausgenommen das Treibmittel, bezogen sind.

25. Kosmetisches Verfahren nach einem vorangehenden Anspruch zum gleichzeitigen Inhibieren des Schwitzens und des Inhibierens des Dunkelwerdens der Haut, das topische Auftragen der Antiperspirant-Zusammensetzung auf die Achsel in einer Menge, die ausreichend ist, um das adstringierende Antiperspirant-Salz in einer Dichte von wenigstens 2 g/m² abzuscheiden, umfasst.

## Revendications

1. Procédé cosmétique permettant d'inhiber ou de prévenir l'assombrissement de la peau provenant de l'application topique à la peau des aisselles d'une composition antitranspirante comprenant un sel antitranspirant actif choisi parmi les sels astringents d'aluminium et/ou de zirconium par incorporation dans la composition d'un système d'inhibition de l'assombrissement comprenant une huile triglycéridique d'acide carboxylique en C₁₈ insaturé, éventuellement avec un acide carboxylique actif choisi parmi
ai) les acides dioïques aliphatiques linéaires contenant de 6 à 12 atomes de carbone ;
aii) les acides carboxyliques en C₁₈ aliphatiques insaturés et
aiii) les acides hydroxybenzoïques,
à un rapport auxdits sels astringents, x/y, de 1/<4 où x représente la concentration en poids de ladite huile triglycéridique plus 4 fois la concentration en poids de l'acide carboxylique actif et y représente la concentration en poids desdits sels astringents.

2. Procédé selon la revendication 1, dans lequel le rapport en poids x/y est de 1/≤3.

3. Procédé selon la revendication 1, dans lequel le rapport en poids x/y est de 2/≤5.

4. Procédé selon l'une quelconque des revendications précédentes, dans lequel le rapport en poids x/y est de 2/≥3.

5. Procédé selon l'une quelconque des revendications précédentes, dans lequel le rapport en poids de l'huile triglycéridique à l'acide carboxylique actif dans la composition est choisi dans la plage pouvant aller jusqu'à 8/1 et est, de préférence, d'au moins 2/1.

6. Procédé selon l'une quelconque des revendications précédentes, dans lequel l'huile triglycéridique comprend l'huile de tournesol, l'huile de ricin ou un mélange de celles-ci.

7. Procédé selon l'une quelconque des revendications précédentes, dans lequel l'acide dicarboxylique aliphatique linéaire est l'acide azélaïque.

8. Procédé selon l'une quelconque des revendications 1 à 5, dans lequel l'acide carboxylique en C₁₈ aliphatique insaturé est un acide linoléique.

9. Procédé selon la revendication 8, dans lequel l'acide linoléique est un acide linoléique conjugué.

10. Procédé selon l'une quelconque des revendications 1 à 5, dans lequel l'acide hydroxybenzoïque est l'acide salicylique.

11. Procédé selon l'une quelconque des revendications précédentes, dans lequel la composition contient de 1 à 10 % en poids d'huile triglycéridique.

12. Procédé selon l'une quelconque des revendications précédentes, dans lequel la composition comprend, en plus, un agent de chélation pour un métal de transition.

13. Procédé selon la revendication 12, dans lequel l'agent de chélation est un acide aminopolycarboxylique.

14. Procédé selon la revendication 13, dans lequel l'acide aminopolycarboxylique est l'acide éthylènediaminetétraacétique ou l'acide di-éthylène-triaminepentaacétique.

15. Procédé selon l'une quelconque des revendications 12 à 14, dans lequel l'agent de chélation est présent à un rapport en poids à l'acide hydroxybenzoïque de 1/20 à 1/5.

16. Procédé selon l'une quelconque des revendications précédentes, dans lequel la composition comprend, en plus, un glycérol.

17. Procédé selon la revendication 16, dans lequel le glycérol est présent dans la composition à un rapport en poids à l'huile triglycéridique de 1/1 à 1/4.

18. Procédé selon l'une quelconque des revendications précédentes, dans lequel la composition contient de 4 à 10 % en poids d'huile triglycéridique et de 0 à moins de 0,5 % d'acide carboxylique actif.

19. Procédé selon l'une quelconque des revendications précédentes, dans lequel la composition contient de 2 à 6 % en poids d'huile triglycéridique et de 0,5 à 2,0 % en poids d'acide carboxylique actif.

20. Procédé selon l'une quelconque des revendications précédentes, dans lequel le sel antitranspirant ou déodorant actif comprend un sel astringent d'aluminium.

21. Procédé selon la revendication 19, dans lequel le sel astringent d'aluminium est un chlorhydrate d'aluminium.

22. Procédé selon l'une quelconque des revendications précédentes, dans lequel le sel antitranspirant ou déodorant actif est présent à une concentration de 10 à 50 % en poids de la composition, exclusion faite de tout gaz propulseur.

23. Procédé selon la revendication 21, dans lequel la composition est dépourvue de gaz propulseur et contient de 10 à 26 % en poids de sel astringent antitranspirant ou déodorant.

24. Procédé selon la revendication 21, dans lequel la composition contient un gaz propulseur et de 20 à 45 % en poids de sel astringent antitranspirant ou déodorant, les % étant basés sur la composition, exclusion faite du gaz propulseur.

25. Procédé cosmétique selon l'une quelconque des revendications précédentes permettant simultanément d'inhiber la transpiration et d'inhiber l'assombrissement de la peau comprenant l'application topique à une aisselle de la composition antitranspirante en une quantité suffisante pour déposer le sel astringent antitranspirant à une densité d'au moins 2 g/m².
